(19)

Europäisches
Patentamt
European
Patent Office
Office européen
des brevets

(11) **EP 4 740 934 A1**

(12) **EUROPEAN PATENT APPLICATION**

(43) Date of publication:
**13.05.2026 Bulletin 2026/20**

(21) Application number: **24211339.7**

(22) Date of filing: **07.11.2024**

(51) International Patent Classification (IPC):
***A61K 8/60*** *(2006.01)*  ***A61Q 5/00*** *(2006.01)*
***A61Q 19/00*** *(2006.01)*

(52) Cooperative Patent Classification (CPC):
**A61Q 5/006; A61K 8/60; A61Q 19/00;**
**A61Q 19/007;** A61K 2800/591

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **SkyLab AG**
**1066 Epalinges (CH)**

(72) Inventors:
• **Belous, Elena**
**121309 MOSCOW (RU)**
• **Ivanova, Angelina**
**LONDON, NW61NE (GB)**

(74) Representative: **Glawe, Delfs, Moll**
**Partnerschaft mbB**
**Hopfenmarkt 33**
**20457 Hamburg (DE)**

(54) **MOISTURIZING AND SCALP MICROBIOME-RESTORING COMPLEX WITH A COMBINATION OF SUGAR DERIVATIVES: LACTOBIONIC ACID, TREHALOSE, AND RHAMNOSE**

(57)    The invention relates to a hair care and/or scalp care composition comprising rhamnose, trehalose and lactobionic acid. Said composition moisturizes the scalp, protects the scalp from dehydration, promotes the growth of *Staphylococcus epidermis* and/or *Cutibacterium acnes* on the scalp and can be used for treating or preventing dandruff.

Hydration index for treatment groups

Fig. 1

EP 4 740 934 A1

**Description**

FIELD OF THE INVENTION

The present invention relates to novel applications of 6-deoxymannose - rhamnose, $\alpha$-D-glucopyranosyl-(1 $\rightarrow$ 1)-$\alpha$-D-glucopyranoside - trehalose, 4-O-$\beta$-galactopyranosyl-D-gluconic acid - lactobionic acid as a medium for promoting scalp moisturization and restoring the commensal microbiome of the scalp. It can be used in the field of hair care products. Rhamnose, trehalose and lactobionic acid are stable and fully suitable for utilization in cosmetic and pharmaceutical formulations.

BACKGROUND ART

The scalp surface represents a specific microenvironment for microorganisms, which is subject to physiologic conditions of the host organism, including cutaneous grease content, humidity and pH. Microbial communities ensure survival on the surface of the host organism, such as the skin, through various regulatory processes, including biofilm formation and quorum definition. Thus, it is expected that metabolic processes between the scalp surface and the microbiome normally support the growth of microbial biofilms in symbiotic, commensal or pathogenic form [1].

Usage of high-throughput sequencing of new generation and robust computational analysis in recent years has led to an in-depth understanding of the scalp microbiome, providing new information regarding the pathophysiology of scalp-related diseases such as dandruff and seborrheic dermatitis. Global investigations have demonstrated that the scalp microbiome is characterized by a rather low bacterial diversity compared to other body areas and is dominated by *Cutibacterium acnes* (formerly *Propionibacterium acnes*), *Staphylococcus epidermidis* and *Malassezia spp. S. epidermidis* and *C. acnes* represent a part of the commensal microbiota and as have been reported make positive impact upon the skin by modulating the immune response and protecting against pathogens. In the investigation [2], the authors conducted a metagenomic analysis of the scalp microflora of an Indian population and have found that healthy scalp compared to scalp covered with dandruff showed an increased content of bacterial pathways associated with the synthesis and metabolism of amino acids, biotin and group B vitamins. This indicated a new potential role for bacterial commensals in maintaining nutrient homeostasis in the scalp: they protect the skin from colonization by pathogens, stimulate production of the complement system and cytokines involved in the initiation and maintenance of the immune response. In addition to this, microbiota helps to reduce inflammation and promotes tissue repair [3].

Since changes in skin physiology are thought to play an important role in shaping the skin microbiome, scalp physiologic parameters such as sebum levels, sunlight exposure, temperature, air humidity, transepidermal moisture loss (TEWL) and hydration levels become particularly important [1,3].

The prevalence of commensal microorganisms on the scalp helps reduce the risk of dandruff, due to the predominance of the fungus *Malassezia spp.* As a result, for the formation of such microbiome, it is important to observe favorable environmental conditions for them, which include the degree of the scalp moisturization. *Propionibacterium spp* lysates have been used as agents to promote scalp moisturization and resulting growth of a commensal scalp microbiome. The lysates can absorb free radicals, preventing damage to cells by free oxygen radicals, thereby reducing inflammation. Due to this, they are used in various anti-inflammatory products [4].

The patent WO2014053625A2 examines the potential of *Propionibacteriumfreudenreichii* lysate to treat and/or prevent scalp diseases by affecting hydration, namely scalp moisturization, scalp barrier function, activating defense mechanisms and immune regulation, thereby reducing scalp inflammation and creating a balanced barrier, preserving its integrity and maintaining a balanced microflora.

However, regulatory considerations and safety issues related to usage of the microbiome in skin care products require focused attention. While oral probiotics have a clear legal definition related to bacteria and yeast, the absence of such a definition for skin probiotics underlines regulatory gaps indicating an urgent need for guidance in this rapidly developing field. Further research is also needed to understand their long-term effects [5].

Various sugars are used as alternative agents that can be used to moisturize the skin. The first example relates to use in oral care and skin care products. Xylitol is a natural alcohol sugar that is known for its effectiveness in reducing caries formation in the oral cavity and it possesses moisturizing properties. The authors of the investigation [6] paid attention to the fact that usage of xylitol (100 mM) during 2 hours improves lipid fluidity in the uppermost layer of the stratum granulosum. Seven volunteers with dry skin showed a 20% reduction in moisture loss when treated with xylitol during 10 minutes compared to water. The effect lasted for two hours after use. The authors suggest that the mechanism is related to the strengthening of tight junctions and barrier formation in the skin. The authors of the investigation [7] evaluated the effect of xylitol on the scalp microbiome. The growth of skin-associated bacteria *Staphylococcus aureus, Staphylococcus epidermidis* and *Cutibacterium acnes* was studied in the presence or absence of 1% (by weight) and 5% (by weight) xylitol. Xylitol inhibited the growth of *S. aureus* and *C. acnes* at 5% concentration while the growth of *S. epidermidis* was stimulated by 1% xylitol but not by 5% xylitol. These results indicate that xylitol has a specific antimicrobial effect and stimulates growth of skin-associated bacteria and can be used in personal care products to control the growth of these bacteria. However, the effect of xylitol on the microbiome was evaluated in-vitro suggesting that clinical studies are needed to evaluate the effects of the component.

Sorbitol is another sugar used in scalp moisturizing products. The authors of the investigation [8] studied skin moisturization in people living in arid and non-arid places, as well as changes in skin moisturization depending on the season. The results of in vitro experiments showed that 50 mM sorbitol protected epidermal keratinocytes from osmotic toxicity induced by sodium chloride. Clinical investigations have shown that skin chronically exposed to hot and dry environments has a stronger skin barrier and lower TEWL baseline. In addition, the skin barrier is stronger in summer than in winter. Sorbitol significantly improved skin barrier repair and moisturization, especially in people living in arid environment conditions. However, there is no research data on the effect of sorbitol on the scalp microbiome.

Trehalose was studied as a component that promotes skin moisturization. This is reflected in the results of investigation [9]. Participants with low water content in the stratum corneum taking part in a randomized, placebo-controlled and double blind study applied a lotion and emulsion containing sodium trehalose sulfate to the face during 4 weeks. Trehalose sodium sulfate reduced transepidermal water loss and increased the water content of the stratum corneum. Patent WO2022008163A1 describes the use of trehalose to regulate the scalp microbiome, in particular the suppression of the scalp pathogen: *Malassezia restricta* [10]. However, the effect on the commenasal scalp microbiome was not disclosed.

The present invention is defined in the claims and relates to a composition comprising the specific combination of trehalose, rhamnose and lactobionic acid, wherein the action of said three components in concert provides for a synergy which could not be expected from the said components alone or in double combination as shown in the following examples. In fact, the applicant is not aware of any prior art disclosure, let alone any evidence in the prior art, suggesting that there may be synergy between the components trehalose, rhamnose and lactobionic acid in terms of hair moisturizing effect and positive influence on the commensal scalp microbiome. As per the applicant's knowledge, said three components have not been studied together previously.

Investigations

Example 1. In vitro determination of the scalp moisturization: hydration and elasticity with complex of Trehalose, Rhamnose and Lactobionic Acid

1.1 Materials and methods

Table 1. Investigated ingredients

| No. | Compound | CAS |
| --- | --- | --- |
| 1 | Rhamnose | 3615-41-6 / 10030-85-0 |
| 2 | Trehalose | 99-20-7 |
| 3 | Lactobionic acid | 96-82-2 |

1.2 Investigation

Subsequent solution preparation protocols: 10 solutions were prepared:

- An isotonic solution containing 0.2% rhamnose in distilled water was prepared. This solution was adjusted to a pH in the range of 5-6, which meets the standards of hair and scalp care products;

- An isotonic solution containing 0.5% trehalose in distilled water was prepared. This solution was adjusted to a pH in the range of 5-6, which is in accordance with the standards of hair and scalp care products;

- An isotonic solution containing 0.5% lactobionic acid in distilled water was prepared. This solution was adjusted to a pH in the range of 5-6, which is in accordance with the standards of hair and scalp care products;

- An isotonic solution containing 0.5% lactobionic acid and 0.2% rhamnose in distilled water was prepared. This solution was adjusted to a pH in the range of 5-6, which is in accordance with the standards of hair and scalp care products;

- An isotonic solution containing 0.5% lactobionic acid and 0.5% trehalose in distilled water was prepared. This solution was adjusted to a pH in the range of 5-6, which is in accordance with the standards of hair and scalp care products;

- An isotonic solution containing 0.5% trehalose and 0.2% rhamnose in distilled water was prepared. This solution was adjusted to a pH in the range of 5-6, which is in accordance with the standards of hair and scalp care products;

- An isotonic solution containing 0.5% lactobionic acid, 0.2% rhamnose and 0.5% trehalose in distilled water was prepared. This solution was adjusted to a pH in the range of 5-6, which is in accordance with the standards of hair and scalp care products;

- An isotonic solution containing 0.1% lactobionic acid, 0.1% rhamnose and 0.1% trehalose in distilled water was prepared. This solution was adjusted to a pH in the range of 5-6, which meets the standards of hair and scalp care products;

- An isotonic solution containing 1.5% lactobionic acid, 0.6% rhamnose and 1.5% trehalose in distilled water was prepared. This solution was adjusted to a pH in the range of 5-6, which meets the standards of hair and scalp care products.

- A distilled water solution was prepared as a negative control.

To measure hydration and elasticity, an ASW (Aramo Smart Wizard) - Aram Human Vision System was used for skin (dermatoscopy) diagnosis under magnification. As a control, the skin was measured for these parameters 20 times. The average value of each parameter was taken for further calculations.

The above solutions were applied to 10 skin areas with a glass rod. The soaking time of the solutions before measurement was 10 minutes. Then each koi section was measured 10 times for each parameter. The mean values were taken for further calculations.

1.3 Results and discussion

Table 2. Hydration index for treatment groups

| No. | SYSTEM | HYDRATION INDEX | STDEV |
|---|---|---|---|
| 1 | Control - untreated skin | 5.6 ($p < 0.05$) | 0.3 |
| 2 | Distilled Water | 6.0 ($p < 0.05$) | 0.4 |
| 3 | 0.2% Rhamnose | 6.0 ($p < 0.05$) | 0.4 |
| 4 | 0.5% Lactobionic Acid | 6.0 ($p < 0.05$) | 0.5 |
| 5 | 0.5% Trehalose | 6.4 ($p < 0.05$) | 0.3 |
| 6 | 0.2% Rhamnose + 0.5% Lactobionic Acid | 5.6 ($p < 0.05$) | 0.5 |
| 7 | 0.2% Rhamnose + 0.5% Trehalose | 6.0 ($p < 0.05$) | 0.4 |
| 8 | 0.5% Lactobionic Acid + 0.5% Trehalose | 7.0 ($p < 0.05$) | 0.3 |
| 9 | 0.5% Lactobionic Acid + 0.5% Trehalose + 0.2% Rhamnose | 7.6 ($p < 0.05$) | 0.3 |
| 10 | 0.1% Lactobionic Acid + 0.1% Trehalose + 0.1% Rhamnose | 7.2 ($p < 0.05$) | 0.3 |
| 11 | 1.5% Lactobionic Acid + 1.5% Trehalose + 0.6% Rhamnose | 8.5 ($p < 0.05$) | 0.4 |

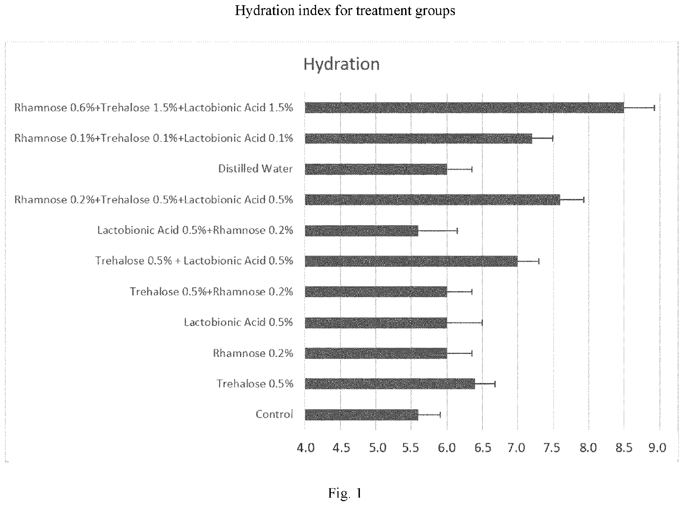

Table 2 and Figure 1 illustrate the results of Hydration index study. The lowest value of this parameter was observed in the control (before any treatment) 5.6 (p<0.05) and in the pair of components lactobionic acid 0.5% and rhamnose 0.2% (5.6(p<0.05)). The use of mono components demonstrated poor efficacy in hydration. For example, lactobionic acid 0.5% improved skin hydration by 7%; rhamnose 0.2% by 7%; trehalose 0.5% by 14%. The paired use of the three components did not demonstrate an improvement in hydration. The use of lactobionic acid 0.5% and rhamnose 0.2%, as previously described, has no effect on the skin compared to the control. The use of rhamnose 0.2% and trehalose 0.5% improves hydration by 7%, which is comparable to the results of the monocomponents. However, a strong efficacy result is demonstrated by the use of lactobionic acid 0.5% and trehalose 0.5%, hydration increases by 25% compared to the control. However, the use of three components together: lactobionic acid, rhamnose and trehalose in the ratio of 0.5:0.2:0.5 shows an unexpected technical result, hydration increases by 36% (7.6 (p<0.05)). The three components demonstrated efficacy over a wide range of concentrations. Using 0.1% of each component increased hydration by 29% compared to the control, higher than using lactobionic acid 0.5% and trehalose 0.5% (hydration increased by 25%), and at lower concentrations. At high concentrations: lactobionic acid 1.5%, rhamnose 0.6% and trehalose 1.5% hydration increases by 52%. Thus, the complex demonstrates efficacy in skin hydration over a wide range of concentrations: specifically from 0.1:0.1:0.1:0.1 to 1.5:0.6: 1.5 for the lactobionic acid, rhamnose and trehalose complex, respectively.

Table 3. Elasticity index for treatment groups

| No. | SYSTEM | ELASTICITY INDEX | STDEV |
|---|---|---|---|
| 1 | Control - untreated skin | 5.6 (p < 0.05) | 0.3 |
| 2 | Distilled Water | 7.2 (p < 0.05) | 0.4 |
| 3 | 0.2% Rhamnose | 7.2 (p < 0.05) | 0.4 |
| 4 | 0.5% Lactobionic Acid | 7.4 (p < 0.05) | 0.6 |
| 5 | 0.5% Trehalose | 7.8 (p < 0.05) | 0.5 |
| 6 | 0.2% Rhamnose + 0.5% Lactobionic Acid | 7.0 (p < 0.05) | 0.7 |
| 7 | 0.2% Rhamnose + 0.5% Trehalose | 7.2 (p < 0.05) | 0.4 |
| 8 | 0.5% Lactobionic Acid + 0.5% Trehalose | 8.6 (p < 0.05) | 0.3 |
| 9 | 0.5% Lactobionic Acid + 0.5% Trehalose + 0.2% Rhamnose | 9.2 (p < 0.05) | 0.4 |
| 10 | 0.1% Lactobionic Acid + 0.1% Trehalose + 0.1% Rhamnose | 8.7 (p < 0.05) | 0.4 |
| 11 | 1,5% Lactobionic Acid + 1,5% Trehalose + 0.6% Rhamnose | 10.2 (p < 0.05) | 0.2 |

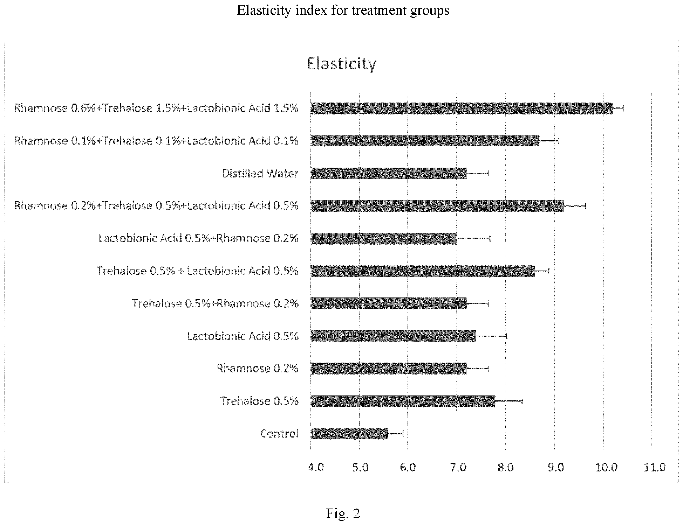

Table 3 and Figure 2 illustrate the results of Hydration index. The lowest value of this parameter was observed in the control (before any treatment) 5.6 (p<0.05). The use of mono components showed comparatively poor efficacy in elasticity. Thus, lactobionic acid 0.5% improved skin elasticity by 32%; rhamnose 0.2% by 29%; trehalose 0.5% by 39%. The paired use of the three components did not demonstrate an improvement in elasticity. The use of lactobionic acid 0.5% and rhamnose 0.2% is comparable in results to the use of the monocomponents, elasticity increases by 25%. The use of rhamnose 0.2% and trehalose 0.5% improves elasticity by 29%, which is also comparable to the results of using monocomponents. However, a strong efficacy result is shown by the use of lactobionic acid 0.5% and trehalose 0.5%, elasticity increases by 54% compared to the control. However, the use of three components together: lactobionic acid, rhamnose and trehalose in the ratio of 0.5:0.2:0.5 shows an unexpected technical result with elasticity increasing by 64% (9.2 (p<0.05)). The three components demonstrated efficacy over a wide range of concentrations. Using 0.1% of each component increases elasticity by 55% compared to the control, which is higher than using lactobionic acid 0.5% and trehalose 0.5% (elasticity increased by 54%), at lower concentrations. At high concentrations: lactobionic acid 1.5%, rhamnose 0.6% and trehalose 1.5% elasticity increases by 82%. Thus, the complex demonstrates efficacy in skin hydration over a wide range of concentrations: specifically from 0.1:0.1:0.1:0.1 to 1.5:0.6:1.5 for lactobionic acid, rhamnose and trehalose complex, respectively.

Thus, the lactobionic acid, rhamnose and trehalose complexes are effective agents for moisturising the skin.

When applied to the skin surface, the complex of lactobionic acid, trehalose and rhamnose increases hydration levels, which may play an important role in shaping the scalp surface microbiome. Thus, it seems reasonable to assume that the application of the described sugars has a strong effect on the physiology of the scalp, which in turn alters the scalp microbiome.

**Example 2**. Evaluation by quantitative PCR of the activity of the substances Lactobionic Acid, Rhamnose and Trehalose against the human scalp microbiota

2.2 Materials and methods

Table 4. Investigated ingredients

| No. | Compound | CAS | Concentration |
|---|---|---|---|
| 1 | Rhamnose | 3615-41-6 / 10030-85-0 | 0.2% |
| 2 | Trehalose | 99-20-7 | 0.5% |
| 3 | Lactobionic acid | 96-82-2 | 0.5% |

Description: transparent solution containing lactobionic acid, rhamnose and trehalose.

Physicochemical characteristics of the components: white powder with solubility ~10 mg/ml in PBS (pH 7.2); ~20 mg/ml in DMSO and DMF (lactobionic acid); white powder, soluble in hot and cold water, molecular weight 182.17, melting point 82°-97°C, specific gravity 1.47 (water 1) (rhamnose); white powder, melting point 203°C, molecular weight 378.1742 (trehalose).

Shelf life: 1 year

Storage conditions: store at a temperature of 0 to +25 °C.

2.2 Investigation

The study protocol was approved by the Local Ethics Committee of the Federal State Autonomous Educational Institution of Higher Education «Kazan Federal University» Ethics Committee for the evaluation of clinical research protocols and was conducted in accordance with the principles outlined in the Declaration of Helsinki of the World Medical Association. Written informed consent was obtained from all participants prior to any procedures related to the study.

2.2.1 Volunteer recruitment conditions

The study recruited 20 people (10 women and 10 men) aged between 18 and 60 years (hereinafter referred to as Study Participants) as volunteers for the study, for whom the following conditions were not applicable:

- Are pregnant or breastfeeding;

- have kidney disease, liver disease, have severe autoimmune diseases in the last 5 years;

- have used perms, keratin straightening, bleaching, permanent perms in the last month preceding the study;

- colouring in the last 14 days preceding the study;

- used antifungal medications/ supplements in the last 60 days preceding the study;

- used anti-dandruff shampoos in the last 60 days preceding the study;

- had a skin disease in the last 3 years prior to the study;

- have taken systemic retinoids orally in the last 60 days prior to the study;

- have used hair loss products in the last 60 days prior to the study.

Study participants complete a questionnaire and have a scalp examination by a dermatologist prior to the study.

2.2.2 Application and collection conditions

On the scalp, two 4×8 cm areas with the most pronounced desquamation were selected. Half of each area (4×4 cm) was sampled with a sterile cotton swab and placed in a microtube with lysis buffer (point 1). Then, the innovative complex of substances (1 ml) was applied to 4×4 cm scalp areas on the other half of the sections using an automatic pipette and left for 3 hours. After 3 hours, a sample was taken from the treated areas with a sterile cotton swab and placed in a microtube with lysing buffer (point 2).

2.2.3 Nucleic acid extraction

Nucleic acids were isolated using LIRA reagent (BioLabMix). A cotton swab, which was used to wash off the head surface, was placed in 1 ml of LIRA reagent and incubated for 10 min at room temperature. Then 200 $\mu$l of chloroform was added to the lysate, the tube was shaken vigorously for 5 s and incubated for 5 min, stirring periodically by hand. Next, the cotton swab was removed and the mixture was centrifuged for 10 min at 10,000 g at +4 °C. After centrifugation, the mixture separated into lower phase (organic), interphase and upper phase (aqueous). The upper phase was transferred to a new eppendorf and RNA was further isolated, while the organic and interphase phases were saved for subsequent DNA isolation.

2.2.3.1 RNA isolation

An equal volume of isopropanol was added to the aqueous phase, the tube was inverted 3 times and incubated for 10 min at room temperature. Then centrifuged for 10 min at 12,000 g at +4 °C. Afterwards, the supernatant was removed and 1 ml of 80% ethanol was added to the precipitate and the tube was inverted 2 times. Then centrifuged for 5 min at 12,000 g at +4 °C, the supernatant was removed. The precipitate was dried for 10 min at room temperature and 15 $\mu$l of RNase-purified water was added. The RNA solution was stored at -20 °C.

2.2.3.2 DNA isolation

The organic phase and interphase were mixed until a homogeneous mixture was obtained and 300 $\mu$l of 96% ethanol was added. The tube was inverted 3 times and incubated for 5 min at room temperature. Then centrifuged for 5 min at 2,000 g at +4 °C. The supernatant was removed and 1 ml of 0.1 M sodium citrate in 10% ethanol (pH 8.5) was added to the precipitate and resuspended. It was incubated for 30 min at room temperature and centrifuged for 5 min at 2,000 g at +4 °C. The supernatant was then removed again and 1 ml of 0.1M sodium citrate in 10% ethanol (pH 8.5) was added to the precipitate and resuspended. Incubated for 30 min at room temperature and centrifuged for 5 min at 2,000 g at +4 °C. 1 ml of 80% ethanol was added to the precipitate and resuspended. Incubated for 20 min, periodically inverting the tube. Centrifuged for 5 min at 2,000 g at +4 °C, the supernatant was removed and the precipitate was dried for 10 min. 15 $\mu$l of 40 mM NaOH was added to the precipitate, and after dissolution, NaOH was neutralised with 0.5 $\mu$l of 1 M HEPES. The DNA solution was stored at -20 °C.

2.2.4 Real-time PCR

PCR was performed with a BioRad CFX96 PCR amplifier ('BioRad', USA) using Biomaster HS-qPCR ('Bio-LabMix'), an extra-mix for quantitative real-time PCR under the conditions recommended by the manufacturer and detection in 4 channels (RO, HEX, FAM, Cy5) for each probe (Table 5).
The 50 $\mu$l reaction mixture contained 1x RT-qPCR buffer, 0.1 $\mu$M of each primer (Table 5), 0.1 $\mu$M of each dNTP, 2.5% DMSO, 5% RT-qPCR extra-mix and sterile water supplemented with DEPC. The 16S rRNA gene for bacteria and the ITS region of 26S rRNA for fungi were used as reference genes.
The PCR mode included a DNA pre-denaturation step at 95 °C for 7 min; then for 40 cycles: denaturation at 95 °C - 30 sec, oligonucleotide annealing at 55 °C - 40 sec, elongation at 72 °C - 35 sec. The annealing temperature of primers was calculated using the Tm Calculator service (https://tmcalculator.neb.com). Real-time PCR monitoring was performed every cycle after the elongation step was completed.
Normalisation was carried out in 2 steps, first we calculated the relative expression of the target gene using the transcription level of the reference gene.

$$\Delta Ct = Ct \text{ (target gene)} - Ct \text{ (reference gene)}$$

Then, the change in the relative content of NK in the samples was determined using the formula:

$$2^{\Delta\Delta Ct} = 2^{\Delta Ct(target\ sample)\ -\ \Delta Ct(reference\ sample)}$$

Table 5. Synthetic oligonucleotides used in the paper [11]

| Primer name | Primer sequence (5'-3') |
|---|---|
| | *C. acnes* |
| CA-P | /ROX/- TTG GAA AGT TTC GGC GGT TG -/BHQ-2/ |
| CA-F | GAC TTT GGG ATA ACT TCA GGA AAC TG |
| CA-R | CTG ATA AGC CGC GAG TCC AT |
| | *S. epidermidis* |
| Se-P | /Cy5/- CTG CTA ATC GTG GTG TTG CTC AAA TTA AA -/BHQ-2/ |
| Se-F | GGA GGA ACT AAT AAT AAG TTA ACT G |
| Se-R | GTC ATA AAC AGT TGT ATA TAA GCC |
| | Universal primers |
| 26S-F | TAA CAA GGA TTC CCC TAG TA |
| 26S-R | ATT ACG CCA GCA TCC TAA G |
| 16S-F | GGG ACC CGC ACA AGC GGT GG |
| 16S-R | GGG TTG CGC TCG TTG CGG GA |

2.2.5 Real-time reverse transcription PCR

PCR was performed using BioRad CFX96 PCR amplifier ('BioRad', USA) with SYBR Blue RT-qPCR ('BioLab-Mix', Russia), an extra-mix for reverse transcription and quantitative real-time one-step PCR using SYBR Green I fluorescent dye under conditions recommended by the manufacturer. The 50 $\mu$l reaction mixture contained 1x SYBR Blue RT-qPCR buffer, 0.1 $\mu$M of each primer (Table 1), 0.1 $\mu$M of each dNTP, 2.5% DMSO, 5% RT-qPCR extra-mix and sterile water supplemented with DEPC. The 16S RNA gene for bacteria and 26S RNA gene for fungi were used as a reference gene.

The OT-PCR mode included a reverse transcription step at 45 °C for 30 min, followed by a DNA pre-denaturation at 95 °C for 7 min; followed by 40 cycles: denaturation at 95 °C for 30 sec, oligonucleotide annealing at 55 °C for 40 sec, and elongation at 72 °C for 35 sec. The annealing temperature of primers was calculated using the Tm Calculator service (https://tmcalculator.neb.com). Real-time PCR monitoring was performed every cycle after the elongation step.

Normalisation was performed in 2 steps, first we calculated the relative expression of the target gene using the transcription level of the reference gene.

$$\Delta Ct = Ct \text{ (target gene)} - Ct \text{ (reference gene)}$$

Then, the change in the relative content of NK in the samples was determined using the formula:

$$2^{\Delta\Delta Ct} = 2^{\Delta Ct(target\ sample) - \Delta Ct(reference\ sample)}$$

2.2.6 Statistical analysis

Microsoft Excel and GraphPad 6.0 software package with a wide range of statistical analysis is used for statistical analysis.

2.3 Results and discussion

Calibration of PCR data

Table 6. CFU counts by intercalator qPCR (*S. epidermidis*)

| qPCR with intercalator (*S. epidermidis*) | |
|---|---|
| Ct (16s) | Log10 (CFU) |
| 14.08 | 9 |
| 19.39 | 8 |
| 21.19 | 7 |
| 23.6 | 6 |
| 24.4 | 5 |
| 26.6 | 4 |
| ND | 3 |
| ND | 2 |

| Ct | Log10 CFUs | CFU/ml |
|---|---|---|
| 20 | 6.81 | 6456542 |
| 25 | 4.625 | 42170 |
| 30 | 2.44 | 275 |
| 35 | 0.255 | 2 |

Table 7. CFU counts by qPCR with probe (*S. epidermidis*)

| qPCR with probe (*S. epidermidis*) | |
|---|---|
| Ct (5s) | Log10 (CFU) |
| 15.23 | 9 |
| 18.01 | 8 |
| 20.25 | 7 |
| 22.45 | 6 |
| 23.93 | 5 |
| 24.74 | 4 |
| 25.3 | 3 |
| ND | 2 |

| Ct | Log10 CFUs | CFU /ml |
|---|---|---|
| 20 | 6.85 | 7079458 |
| 25 | 4.155 | 14289 |
| 30 | 1.46 | 29 |
| 35 | -1.235 | 0 |

Relative DNA content of *Staphylococcus epidermidis* in samples.

Table 8. Calculation of the relative DNA content of *Staphylococcus epidermidis* in samples

| Sample No. | Before treatment | | | After treatment | | | |
|---|---|---|---|---|---|---|---|
| | probe | 16s | ΔCt | probe | 16s | ΔCt | 2$^{\Delta\Delta Ct}$ |
| | | | | | | | |

**First repeat**

| Sample No. | probe | 16s | ΔCt | probe | 16s | ΔCt | 2$^{\Delta\Delta Ct}$ |
|---|---|---|---|---|---|---|---|
| 1 | 14 | 25 | -11 | 16 | 26 | -10 | 0.500 |
| 2 | 14 | 26 | -12 | 14 | 25 | -11 | 0.500 |
| 3 | 13 | 25 | -12 | 13 | 26 | -13 | 2.000 |
| 4 | 12 | 25 | -13 | 18 | 26 | -8 | 0.042 |
| 5 | 20 | 25 | -5 | 14 | 26 | -12 | 171.598 |
| 6 | 13 | 26 | -13 | 13 | 26 | -13 | 1.070 |
| 7 | 14 | 25 | -11 | 14 | 24 | -10 | 0.500 |
| 8 | 13 | 25 | -12 | 12 | 24 | -12 | 1.000 |
| 9 | 13 | 25 | -12 | 12 | 24 | -12 | 1.000 |
| 10 | 11 | 25 | -14 | 12 | 25 | -13 | 0.500 |
| 11 | 13 | 25 | -12 | 13 | 25 | -12 | 1.065 |
| 12 | 14 | 25 | -11 | 14 | 25 | -11 | 1.000 |
| 13 | 12 | 26 | -14 | 16 | 28 | -12 | 0.250 |
| 14 | 14 | 27 | -13 | 13 | 28 | -15 | 4.000 |
| 15 | 12 | 28 | -16 | 13 | 28 | -15 | 0.500 |
| 16 | 11 | 27 | -16 | 13 | 27 | -14 | 0.250 |
| 17 | 12 | 28 | -16 | 12 | 28 | -16 | 0.863 |
| 18 | 12 | 28 | -16 | 13 | 27 | -14 | 0.250 |
| 19 | 15 | 26 | -11 | 10 | 25 | -15 | 16.000 |
| 20 | 12 | 27 | -15 | 13 | 27 | -14 | 0.500 |
| 21 | 13 | 26 | -13 | 14 | 28 | -14 | 2.000 |
| 22 | 13 | 27 | -14 | 13 | 28 | -15 | 2.000 |
| 23 | 11 | 24 | -13 | 11 | 24 | -13 | 1.000 |
| 24 | 13 | 28 | -15 | 12 | 27 | -15 | 1.000 |

**Second repeat**

| Sample No. | probe | 16s | ΔCt | probe | 16s | ΔCt | 2$^{\Delta\Delta Ct}$ |
|---|---|---|---|---|---|---|---|
| 1 | 17 | 26 | -9 | 17 | 27 | -10 | 2.000 |
| 2 | 15 | 27 | -12 | 15 | 26 | -11 | 0.500 |
| 3 | 14 | 26 | -12 | 14 | 30 | -16 | 16.000 |
| 4 | 14 | 26 | -12 | 14 | 26 | -12 | 1.000 |
| 5 | 12 | 27 | -15 | 13 | 27 | -14 | 0.484 |
| 6 | 14 | 26 | -12 | 14 | 26 | -12 | 1.000 |
| 7 | 14 | 27 | -13 | 18 | 25 | -7 | 0.016 |
| 8 | 14 | 26 | -12 | 13 | 25 | -12 | 1.000 |
| 9 | 15 | 26 | -11 | 13 | 25 | -12 | 1.898 |
| 10 | 13 | 25 | -12 | 13 | 26 | -13 | 2.000 |

(continued)

| Second repeat | | | | | | |
|---|---|---|---|---|---|---|
| Sample No. | Before treatment | | | After treatment | | |
| | probe | 16s | ΔCt | probe | 16s | ΔCt | $2^{\Delta\Delta Ct}$ |
| 11 | 15 | 26 | -11 | 13 | 25 | -12 | 2.000 |
| 12 | 13 | 25 | -12 | 16 | 26 | -10 | 0.250 |
| 13 | 12 | 26 | -14 | 12 | 29 | -17 | 8.000 |
| 14 | 15 | 27 | -12 | 15 | 28 | -13 | 2.000 |
| 15 | 14 | 28 | -14 | 14 | 28 | -14 | 1.000 |
| 16 | 14 | 27 | -13 | 14 | 26 | -12 | 0.500 |
| 17 | 14 | 27 | -13 | 14 | 27 | -13 | 1.000 |
| 18 | 14 | 28 | -14 | 14 | 27 | -13 | 0.500 |
| 19 | 14 | 26 | -12 | 12 | 25 | -13 | 2.000 |
| 20 | 20 | 27 | -7 | 20 | 27 | -7 | 1.000 |
| 21 | 14 | 26 | -12 | 12 | 27 | -15 | 8.000 |
| 22 | 13 | 27 | -14 | 13 | 28 | -15 | 2.000 |
| 23 | 12 | 24 | -12 | 13 | 24 | -11 | 0.500 |
| 24 | 13 | 27 | -14 | 6 | 26 | -20 | 64.000 |
| Third repeat | | | | | | |
| Sample No. | Before treatment | | | After treatment | | |
| | probe | 16s | ΔCt | probe | 16s | ΔCt | $2^{\Delta\Delta Ct}$ |
| 1 | 19 | 27 | -8 | 20 | 24 | -4 | 0.063 |
| 2 | 13 | 26 | -13 | 17 | 24 | -7 | 0.018 |
| 3 | 19 | 26 | -7 | 19 | 28 | -9 | 4.000 |
| 4 | 17 | 26 | -9 | 16 | 27 | -11 | 4.000 |
| 5 | 13 | 27 | -14 | 12 | 26 | -14 | 1.000 |
| 6 | 15 | 26 | -11 | 17 | 25 | -8 | 0.125 |
| 7 | 16 | 26 | -10 | 16 | 25 | -9 | 0.411 |
| 8 | 13 | 23 | -10 | 13 | 26 | -13 | 8.000 |
| 9 | 17 | 26 | -9 | 14 | 27 | -13 | 16.000 |
| 10 | 13 | 26 | -13 | 15 | 28 | -13 | 0.831 |
| 11 | 16 | 27 | -11 | 14 | 25 | -11 | 1.357 |
| 12 | 19 | 25 | -6 | 17 | 26 | -9 | 6.454 |
| 13 | 10 | 27 | -17 | 11 | 23 | -12 | 0.031 |
| 14 | 12 | 27 | -15 | 13 | 27 | -14 | 0.500 |
| 15 | 11 | 28 | -17 | 14 | 28 | -14 | 0.125 |
| 16 | 11 | 27 | -16 | 11 | 26 | -15 | 0.500 |
| 17 | 14 | 28 | -14 | 14 | 28 | -14 | 1.000 |
| 18 | 12 | 27 | -15 | 13 | 27 | -14 | 0.500 |
| 19 | 10 | 25 | -15 | 11 | 25 | -14 | 0.500 |
| 20 | 12 | 27 | -15 | 13 | 27 | -14 | 0.500 |

(continued)

| Third repeat | | | | | | | |
|---|---|---|---|---|---|---|---|
| Sample No. | Before treatment | | | After treatment | | | |
| | probe | 16s | ΔCt | probe | 16s | ΔCt | $2^{\Delta\Delta Ct}$ |
| 21 | 12 | 26 | -14 | 12 | 27 | -15 | 2.000 |
| 22 | 11 | 27 | -16 | 11 | 28 | -17 | 2.000 |
| 23 | 8 | 24 | -16 | 9 | 23 | -14 | 0.250 |

As a result of exposure to the complex of lactobionic acid 0.5%, rhamnose 0.2% and trehalose 0.5%, an increase in the number of DNA copies of *Staphylococcus epidermidis* by $2.9 \pm 4.5$ times was observed. Thus, the use of the complex has a positive effect on the commensal microbiota of the scalp, in particular on *Staphylococcus epidermidis,* as their numbers increase.

Relative content of *Cutibacterium acnes* DNA in samples.

Table 9. Calculation of the relative DNA content of *Cutibacterium acnes* in samples

| First repeat | | | | | | | |
|---|---|---|---|---|---|---|---|
| Sample No. | Before treatment | | | After treatment | | | |
| | probe | 16s | ΔCt | probe | 16s | ΔCt | $2^{\Delta\Delta Ct}$ |
| 1 | 0 | 25 | -25 | 0 | 26 | -26 | 2.000 |
| 2 | 31 | 26 | 5 | 25 | 25 | 0 | 32.000 |
| 3 | 27 | 25 | 2 | 27 | 26 | 1 | 2.000 |
| 4 | 27 | 25 | 2 | 0 | 26 | -26 | 268435456.000 |
| 5 | 23 | 25 | -2 | 24 | 26 | -2 | 1.000 |
| 6 | 0 | 26 | -26 | 27 | 26 | 1 | 0.000 |
| 7 | 30 | 25 | 5 | 29 | 24 | 5 | 1.000 |
| 8 | 23 | 25 | -2 | 23 | 24 | -1 | 0.500 |
| 9 | 30 | 25 | 5 | 26 | 24 | 2 | 8.000 |
| 10 | 25 | 25 | 0 | 25 | 25 | 0 | 1.000 |
| 11 | 26 | 25 | 1 | 25 | 25 | 0 | 2.000 |
| 12 | 27 | 25 | 2 | 27 | 25 | 2 | 1.000 |
| 13 | 29 | 26 | 3 | 29 | 28 | 1 | 4.000 |
| 14 | 31 | 27 | 4 | 29 | 28 | 1 | 8.000 |
| 15 | 27 | 28 | -1 | 30 | 28 | 2 | 0.125 |
| 16 | 26 | 27 | -1 | 27 | 27 | 0 | 0.500 |
| 17 | 30 | 28 | 2 | 31 | 28 | 3 | 0.500 |
| 18 | 31 | 28 | 3 | 30 | 27 | 3 | 1.000 |
| 19 | 28 | 26 | 2 | 26 | 25 | 1 | 2.000 |
| 20 | 28 | 27 | 1 | 28 | 27 | 1 | 1.000 |
| 21 | 29 | 26 | 3 | 28 | 28 | 0 | 8.000 |
| 22 | 29 | 27 | 2 | 29 | 28 | 1 | 2.000 |
| 23 | 22 | 24 | -2 | 22 | 24 | -2 | 1.000 |
| 24 | 27 | 28 | -1 | 25 | 27 | -2 | 2.000 |

(continued)

| Second repeat | | | | | | | |
|---|---|---|---|---|---|---|---|
| Sample No. | Before treatment | | | After treatment | | | |
| | probe | 16s | ΔCt | probe | 16s | ΔCt | $2^{\Delta\Delta Ct}$ |
| 1 | 31 | 26 | 5 | 31 | 27 | 4 | 2.000 |
| 2 | 28 | 27 | 1 | 24 | 26 | -2 | 8.000 |
| 3 | 27 | 26 | 1 | 29 | 30 | -1 | 4.000 |
| 4 | 27 | 26 | 1 | 0 | 26 | -26 | 134217728.000 |
| 5 | 23 | 27 | -4 | 24 | 27 | -3 | 0.500 |
| 6 | 29 | 26 | 3 | 26 | 26 | 0 | 8.000 |
| 7 | 28 | 27 | 1 | 28 | 25 | 3 | 0.250 |
| 8 | 24 | 26 | -2 | 23 | 25 | -2 | 1.000 |
| 9 | 29 | 26 | 3 | 25 | 25 | 0 | 8.000 |
| 10 | 25 | 25 | 0 | 24 | 26 | -2 | 4.000 |
| 11 | 26 | 26 | 0 | 24 | 25 | -1 | 2.000 |
| 12 | 26 | 25 | 1 | 27 | 26 | 1 | 1.000 |
| 13 | 28 | 26 | 2 | 28 | 28 | 0 | 4.000 |
| 14 | 30 | 27 | 3 | 28 | 28 | 0 | 8.000 |
| 15 | 27 | 28 | -1 | 20 | 28 | -8 | 128.000 |
| 16 | 26 | 27 | -1 | 26 | 26 | 0 | 0.500 |
| 17 | 30 | 27 | 3 | 29 | 27 | 2 | 2.000 |
| 18 | 31 | 28 | 3 | 30 | 27 | 3 | 1.000 |
| 19 | 29 | 26 | 3 | 23 | 25 | -2 | 32.000 |
| 20 | 27 | 27 | 0 | 29 | 27 | 2 | 0.250 |
| 21 | 0 | 26 | -26 | 28 | 27 | 1 | 0.000 |
| 22 | 30 | 27 | 3 | 29 | 28 | 1 | 4.000 |
| 23 | 21 | 24 | -3 | 21 | 24 | -3 | 1.000 |
| 24 | 27 | 27 | 0 | 24 | 26 | -2 | 4.000 |
| Third repeat | | | | | | | |
| Sample No. | Before treatment | | | After treatment | | | |
| | probe | 16s | ΔCt | probe | 16s | ΔCt | $2^{\Delta\Delta Ct}$ |
| 1 | 31 | 27 | 4 | 32 | 27 | 5 | 0.500 |
| 2 | 29 | 26 | 3 | 24 | 26 | -2 | 32.000 |
| 3 | 26 | 26 | 0 | 27 | 26 | 1 | 0.500 |
| 4 | 26 | 26 | 0 | 0 | 27 | -27 | 134217728.000 |
| 5 | 24 | 27 | -3 | 25 | 26 | -1 | 0.250 |
| 6 | 29 | 26 | 3 | 32 | 25 | 7 | 0.063 |
| 7 | 29 | 26 | 3 | 28 | 25 | 3 | 1.000 |
| 8 | 24 | 23 | 1 | 24 | 26 | -2 | 8.000 |
| 9 | 28 | 26 | 2 | 26 | 27 | -1 | 8.000 |
| 10 | 24 | 26 | -2 | 24 | 28 | -4 | 4.000 |

(continued)

| Third repeat | | | | | | | |
|---|---|---|---|---|---|---|---|
| Sample No. | Before treatment | | | After treatment | | | |
| | probe | 16s | ΔCt | probe | 16s | ΔCt | $2^{\Delta\Delta Ct}$ |
| 11 | 26 | 27 | -1 | 24 | 25 | -1 | 1.000 |
| 12 | 26 | 25 | 1 | 27 | 26 | 1 | 1.000 |
| 13 | 27 | 27 | 0 | 27 | 23 | 4 | 0.063 |
| 14 | 28 | 27 | 1 | 26 | 27 | -1 | 4.000 |
| 15 | 26 | 28 | -2 | 29 | 28 | 1 | 0.125 |
| 16 | 26 | 27 | -1 | 26 | 26 | 0 | 0.500 |
| 17 | 29 | 28 | 1 | 29 | 28 | 1 | 1.000 |
| 18 | 30 | 27 | 3 | 30 | 27 | 3 | 1.000 |
| 19 | 26 | 25 | 1 | 23 | 25 | -2 | 8.000 |
| 20 | 27 | 27 | 0 | 28 | 27 | 1 | 0.500 |
| 21 | 29 | 26 | 3 | 27 | 27 | 0 | 8.000 |
| 22 | 28 | 27 | 1 | 27 | 28 | -1 | 4.000 |
| 23 | 21 | 24 | -3 | 20 | 23 | -3 | 1.000 |
| 24 | 26 | 28 | -2 | 24 | 26 | -2 | 1.000 |
| 24 | 12 | 28 | -16 | 12 | 26 | -14 | 0.250 |

Because of exposure to the complex of lactobionic acid 0.5%, rhamnose 0.2% and trehalose 0.5%, an increase in the number of DNA copies of *Cutibacterium acnes* by 3.9±5.5 times was observed. Thus, the use of the complex has a positive effect on the commensal microbiota of the scalp, in particular on *Cutibacterium acnes,* as their numbers increase.
Relative RNA content of *Staphylococcus epidermidis* in samples.

Table 10. Calculation of the relative RNA content of *Staphylococcus epidermidis* in samples

| First repeat | | | | | | | |
|---|---|---|---|---|---|---|---|
| Sample No. | Before treatment | | | After treatment | | | |
| | probe | 16s | ΔCt | probe | 16s | ΔCt | $2^{\Delta\Delta Ct}$ |
| 1 | 37 | 27 | 10 | 20 | 28 | -8 | 367082.658 |
| 2 | 20 | 24 | -4 | 20 | 27 | -7 | 8.000 |
| 3 | 37 | 22 | 15 | 34 | 22 | 12 | 8.000 |
| 4 | 40 | 21 | 19 | 39 | 23 | 16 | 11.146 |
| 5 | 36 | 21 | 15 | 38 | 22 | 16 | 0.661 |
| 6 | 40 | 24 | 16 | 40 | 23 | 17 | 0.500 |
| 7 | 39 | 23 | 16 | 39 | 24 | 15 | 2.000 |
| 8 | 38 | 29 | 9 | 38 | 27 | 11 | 0.250 |
| 9 | 40 | 22 | 18 | 40 | 23 | 17 | 1.787 |
| 10 | 39 | 24 | 15 | 39 | 23 | 16 | 0.452 |
| 11 | 39 | 23 | 16 | 39 | 22 | 17 | 0.500 |
| 12 | 40 | 22 | 18 | 40 | 21 | 19 | 0.435 |
| 13 | 10 | 22 | -12 | 7 | 22 | -15 | 8.000 |
| 14 | 10 | 26 | -16 | 11 | 22 | -11 | 0.031 |

(continued)

| First repeat | | | | | | |
|---|---|---|---|---|---|---|
| Sample No. | Before treatment | | | After treatment | | |
| | probe | 16s | ΔCt | probe | 16s | ΔCt | $2^{\Delta\Delta Ct}$ |
| 15 | 41 | 27 | 14 | 43 | 25 | 18 | 0.063 |
| 16 | 6 | 23 | -17 | 12 | 20 | -8 | 0.002 |
| 17 | 8 | 21 | -13 | 10 | 21 | -11 | 0.250 |
| 18 | 8 | 22 | -14 | 8 | 20 | -12 | 0.250 |
| 19 | 8 | 22 | -14 | 10 | 23 | -13 | 0.500 |
| 20 | 6 | 21 | -15 | 8 | 23 | -15 | 1.000 |
| 21 | 12 | 26 | -14 | 5 | 25 | -20 | 64.000 |
| 22 | 13 | 22 | -9 | 7 | 23 | -16 | 128.000 |
| 23 | 14 | 22 | -8 | 8 | 22 | -14 | 64.000 |
| 24 | 11 | 22 | -11 | 11 | 21 | -10 | 0.500 |

| Second repeat | | | | | | |
|---|---|---|---|---|---|---|
| Sample No. | Before treatment | | | After treatment | | |
| | probe | 16s | ΔCt | probe | 16s | ΔCt | $2^{\Delta\Delta Ct}$ |
| 1 | 37 | 26 | 11 | 44 | 27 | 17 | 0.016 |
| 2 | 20 | 24 | -4 | 0 | 26 | -26 | 4194304.000 |
| 3 | 37 | 23 | 14 | 34 | 23 | 11 | 8.000 |
| 4 | 30 | 24 | 6 | 30 | 25 | 5 | 2.000 |
| 5 | 36 | 23 | 13 | 36 | 24 | 12 | 2.000 |
| 6 | 39 | 25 | 14 | 32 | 25 | 7 | 128.000 |
| 7 | 38 | 25 | 13 | 38 | 25 | 13 | 1.000 |
| 8 | 0 | 28 | -28 | 40 | 26 | 14 | 0.000 |
| 9 | 36 | 24 | 12 | 38 | 22 | 16 | 0.063 |
| 10 | 39 | 24 | 15 | 40 | 24 | 16 | 0.500 |
| 11 | 39 | 24 | 15 | 39 | 24 | 15 | 0.732 |
| 12 | 40 | 24 | 16 | 38 | 23 | 15 | 2.000 |
| 13 | 12 | 22 | -10 | 6 | 23 | -17 | 128.000 |
| 14 | 40 | 25 | 15 | 40 | 22 | 18 | 0.125 |
| 15 | 9 | 26 | -17 | 11 | 25 | -14 | 0.125 |
| 16 | 9 | 22 | -13 | 8 | 19 | -11 | 0.250 |
| 17 | 10 | 21 | -11 | 8 | 21 | -13 | 4.000 |
| 18 | 8 | 21 | -13 | 13 | 25 | -12 | 0.500 |
| 19 | 12 | 22 | -10 | 9 | 22 | -13 | 8.000 |
| 20 | 7 | 21 | -14 | 5 | 22 | -17 | 8.000 |
| 21 | 13 | 25 | -12 | 13 | 24 | -11 | 0.500 |
| 22 | 44 | 20 | 24 | 45 | 21 | 24 | 1.000 |
| 23 | 37 | 23 | 14 | 43 | 22 | 21 | 0.008 |
| 24 | 15 | 22 | -7 | 11 | 22 | -11 | 16.000 |

(continued)

| Third repeat | | | | | | | |
|---|---|---|---|---|---|---|---|
| Sample No. | Before treatment | | | After treatment | | | |
| | probe | 16s | ΔCt | probe | 16s | ΔCt | $2^{ΔΔCt}$ |
| 1 | 37 | 26 | 11 | 42 | 28 | 14 | 0.125 |
| 2 | 39 | 25 | 14 | 44 | 26 | 18 | 0.063 |
| 3 | 45 | 24 | 21 | 37 | 25 | 12 | 512.000 |
| 4 | 0 | 25 | -25 | 44 | 25 | 19 | 0.000 |
| 5 | 40 | 26 | 14 | 37 | 24 | 13 | 2.000 |
| 6 | 37 | 22 | 15 | 41 | 25 | 16 | 0.500 |
| 7 | 38 | 25 | 13 | 40 | 25 | 15 | 0.250 |
| 8 | 37 | 28 | 9 | 39 | 26 | 13 | 0.054 |
| 9 | 32 | 24 | 8 | 37 | 24 | 13 | 0.031 |
| 10 | 19 | 25 | -6 | 39 | 25 | 14 | 0.000 |
| 11 | 42 | 25 | 17 | 40 | 23 | 17 | 1.000 |
| 12 | 33 | 32 | 1 | 45 | 25 | 20 | 0.000 |
| 13 | 40 | 21 | 19 | 44 | 23 | 21 | 0.250 |
| 14 | 42 | 24 | 18 | 43 | 22 | 21 | 0.125 |
| 15 | 38 | 25 | 13 | 44 | 24 | 20 | 0.008 |
| 16 | 48 | 24 | 24 | 49 | 19 | 30 | 0.016 |
| 17 | 21 | 21 | 0 | 20 | 21 | -1 | 2.000 |
| 18 | 42 | 21 | 21 | 46 | 24 | 22 | 0.500 |
| 19 | 20 | 21 | -1 | 20 | 24 | -4 | 8.000 |
| 20 | 19 | 21 | -2 | 17 | 22 | -5 | 8.000 |
| 21 | 18 | 24 | -6 | 18 | 23 | -5 | 0.500 |
| 22 | 18 | 21 | -3 | 16 | 22 | -6 | 8.000 |
| 23 | 14 | 20 | -6 | 8 | 20 | -12 | 64.000 |
| 24 | 9 | 21 | -12 | 10 | 19 | -9 | 0.125 |

As a result of exposure to the complex of lactobionic acid 0.5%, rhamnose 0.2% and trehalose 0.5%, an increase in the number of RNA copies of *Staphylococcus epidermidis* by 17±38 times was observed. This indicates active growth, or increased availability of these bacteria for detection methods. Thus, the use of the complex has a positive effect on the commensal microbiota of the scalp, in particular on *Staphylococcus epidermidis,* as their numbers increase.

Relative RNA content of *Cutibacterium acnes* in samples.

Table 11. Calculation of the relative RNA content of *Cutibacterium acnes* in samples

| First repeat | | | | | | | |
|---|---|---|---|---|---|---|---|
| Sample No. | Before treatment | | | After treatment | | | |
| | probe | 16s | ΔCt | probe | 16s | ΔCt | $2^{ΔΔCt}$ |
| 1 | 27 | 26 | 1 | 31 | 28 | 3 | 0.188 |
| 2 | 26 | 24 | 2 | 26 | 27 | -1 | 5.854 |
| 3 | 24 | 22 | 2 | 23 | 22 | 1 | 2.000 |
| 4 | 24 | 21 | 3 | 23 | 23 | 0 | 11.146 |

(continued)

| First repeat | | | | | | |
|---|---|---|---|---|---|---|
| Sample No. | Before treatment | | | After treatment | | |
| | probe | 16s | $\Delta$Ct | probe | 16s | $\Delta$Ct | $2^{\Delta\Delta Ct}$ |
| 5 | 21 | 21 | 0 | 22 | 22 | 0 | 1.323 |
| 6 | 25 | 24 | 1 | 24 | 23 | 1 | 1.000 |
| 7 | 25 | 23 | 2 | 25 | 24 | 1 | 2.000 |
| 8 | 26 | 29 | -3 | 23 | 27 | -4 | 2.000 |
| 9 | 25 | 22 | 3 | 22 | 23 | -1 | 14.296 |
| 10 | 21 | 24 | -3 | 23 | 23 | 0 | 0.113 |
| 11 | 24 | 23 | 1 | 21 | 22 | -1 | 4.000 |
| 12 | 24 | 22 | 2 | 23 | 21 | 2 | 0.870 |
| 13 | 26 | 22 | 4 | 26 | 22 | 4 | 1.000 |
| 14 | 26 | 26 | 0 | 26 | 22 | 4 | 0.063 |
| 15 | 25 | 27 | -2 | 25 | 25 | 0 | 0.250 |
| 16 | 26 | 23 | 3 | 25 | 20 | 5 | 0.250 |
| 17 | 26 | 21 | 5 | 26 | 21 | 5 | 1.000 |
| 18 | 27 | 22 | 5 | 27 | 20 | 7 | 0.250 |
| 19 | 23 | 22 | 1 | 24 | 23 | 1 | 1.000 |
| 20 | 26 | 21 | 5 | 26 | 23 | 3 | 4.000 |
| 21 | 27 | 26 | 1 | 26 | 25 | 1 | 1.000 |
| 22 | 26 | 22 | 4 | 26 | 23 | 3 | 2.000 |
| 23 | 22 | 22 | 0 | 21 | 22 | -1 | 2.000 |
| 24 | 24 | 22 | 2 | 22 | 21 | 1 | 2.000 |

| Second repeat | | | | | | |
|---|---|---|---|---|---|---|
| Sample No. | Before treatment | | | After treatment | | |
| | probe | 16s | $\Delta$Ct | probe | 16s | $\Delta$Ct | $2^{\Delta\Delta Ct}$ |
| 1 | 25 | 26 | -1 | 26 | 27 | -1 | 1.327 |
| 2 | 24 | 24 | 0 | 24 | 26 | -2 | 4.000 |
| 3 | 24 | 23 | 1 | 23 | 23 | 0 | 2.000 |
| 4 | 24 | 24 | 0 | 25 | 25 | 0 | 1.000 |
| 5 | 22 | 23 | -1 | 22 | 24 | -2 | 2.000 |
| 6 | 24 | 25 | -1 | 24 | 25 | -1 | 1.000 |
| 7 | 26 | 25 | 1 | 25 | 25 | 0 | 2.000 |
| 8 | 25 | 28 | -3 | 23 | 26 | -3 | 1.207 |
| 9 | 24 | 24 | 0 | 22 | 22 | 0 | 1.000 |
| 10 | 23 | 24 | -1 | 23 | 24 | -1 | 1.000 |
| 11 | 25 | 24 | 1 | 22 | 24 | -2 | 5.852 |
| 12 | 25 | 24 | 1 | 24 | 23 | 1 | 1.000 |
| 13 | 23 | 22 | 1 | 24 | 23 | 1 | 1.000 |
| 14 | 24 | 25 | -1 | 24 | 22 | 2 | 0.125 |

(continued)

| Second repeat | | | | | | |
|---|---|---|---|---|---|---|
| Sample No. | Before treatment | | | After treatment | | |
| | probe | 16s | $\Delta$Ct | probe | 16s | $\Delta$Ct | $2^{\Delta\Delta Ct}$ |
| 15 | 24 | 26 | -2 | 24 | 25 | -1 | 0.500 |
| 16 | 25 | 22 | 3 | 23 | 19 | 4 | 0.500 |
| 17 | 24 | 21 | 3 | 25 | 21 | 4 | 0.500 |
| 18 | 25 | 21 | 4 | 24 | 25 | -1 | 32.000 |
| 19 | 23 | 22 | 1 | 23 | 22 | 1 | 1.000 |
| 20 | 25 | 21 | 4 | 24 | 22 | 2 | 4.000 |
| 21 | 25 | 25 | 0 | 24 | 24 | 0 | 1.000 |
| 22 | 22 | 20 | 2 | 21 | 21 | 0 | 4.000 |
| 23 | 24 | 23 | 1 | 22 | 22 | 0 | 2.000 |
| 24 | 24 | 22 | 2 | 23 | 22 | 1 | 2.000 |
| Third repeat | | | | | | |
| Sample No. | Before treatment | | | After treatment | | |
| | probe | 16s | $\Delta$Ct | probe | 16s | $\Delta$Ct | $2^{\Delta\Delta Ct}$ |
| 1 | 29 | 26 | 3 | 31 | 28 | 3 | 1.000 |
| 2 | 29 | 25 | 4 | 28 | 26 | 2 | 4.000 |
| 3 | 27 | 24 | 3 | 27 | 25 | 2 | 2.000 |
| 4 | 29 | 25 | 4 | 28 | 25 | 3 | 1.639 |
| 5 | 26 | 26 | 0 | 26 | 24 | 2 | 0.343 |
| 6 | 28 | 22 | 6 | 28 | 25 | 3 | 8.000 |
| 7 | 28 | 25 | 3 | 28 | 25 | 3 | 1.000 |
| 8 | 28 | 28 | 0 | 27 | 26 | 1 | 0.429 |
| 9 | 28 | 24 | 4 | 25 | 24 | 1 | 8.000 |
| 10 | 23 | 25 | -2 | 26 | 25 | 1 | 0.152 |
| 11 | 28 | 25 | 3 | 26 | 23 | 3 | 1.000 |
| 12 | 28 | 32 | -4 | 28 | 25 | 3 | 0.008 |
| 13 | 23 | 21 | 2 | 24 | 23 | 1 | 2.000 |
| 14 | 24 | 24 | 0 | 24 | 22 | 2 | 0.250 |
| 15 | 24 | 25 | -1 | 24 | 24 | 0 | 0.500 |
| 16 | 25 | 24 | 1 | 23 | 19 | 4 | 0.125 |
| 17 | 24 | 21 | 3 | 24 | 21 | 3 | 1.000 |
| 18 | 25 | 21 | 4 | 25 | 24 | 1 | 8.000 |
| 19 | 23 | 21 | 2 | 24 | 24 | 0 | 4.000 |
| 20 | 24 | 21 | 3 | 24 | 22 | 2 | 2.000 |
| 21 | 25 | 24 | 1 | 24 | 23 | 1 | 1.000 |
| 22 | 25 | 21 | 4 | 25 | 22 | 3 | 2.000 |
| 23 | 21 | 20 | 1 | 20 | 20 | 0 | 2.000 |
| 24 | 23 | 21 | 2 | 20 | 19 | 1 | 2.000 |

As a result of exposure to the complex of lactobionic acid 0.5%, rhamnose 0.2% and trehalose 0.5%, an increase in the number of RNA copies of *Cutibacterium acnes* by 2.9±4 times was observed. This indicates active growth, or increased availability of these bacteria for detection methods. Thus, the use of the complex has a positive effect on the commensal microbiota of the scalp. in particular on *Cutibacterium acnes.* as their numbers increase.

Thus. the complex consisting of lactobionic acid, rhamnose and trehalose has a positive effect on the commensal microbiome of the scalp.

Literature

1. Saxena R. Mittal P. Clavaud C. Dhakan DB. Hegde P. Veeranagaiah MM. Saha S. Souverain L. Roy N. Breton L. Misra N. Sharma VK. Comparison of Healthy and Dandruff Scalp Microbiome Reveals the Role of Commensals in Scalp Health. Front Cell Infect Microbiol. 2018 Oct 4;8:346. doi: 10.3389/fcimb.2018.00346. PMID: 30338244; PMCID: PMC6180232.

2. Saxena. R.. Mittal. P.. Clavaud. C. et al. Longitudinal study of the scalp microbiome suggests coconut oil to enrich healthy scalp commensals. Sci Rep 11. 7220 (2021). https://doi.org/10.1038/s41598-021-86454-1

3. Polak-Witka K. Rudnicka L. Blume-Peytavi U. Vogt A. The role of the microbiome in scalp hair follicle biology and disease. Exp Dermatol. 2020 Mar;29(3):286-294. doi: 10.1111/exd. 13935. Epub 2019 May 15. PMID: 30974503.

4. Gallo G. Grossi U. Di Tanna GL. Santoro GA. De Paola G. Clerico G. Realis Luc A. Trompetto M. Sammarco G. Short-Term Outcomes of Polycarbophil and Propionibacterium acnes Lysate Gel after Open Hemorrhoidectomy: A Prospective Cohort Study. J Clin Med. 2020 Dec 10;9(12):3996. doi: 10.3390/jcm9123996. PMID: 33321707; PMCID: PMC7763882.

5. Al-Smadi K. Leite-Silva VR. Filho NA. Lopes PS. Mohammed Y. Innovative Approaches for Maintaining and Enhancing Skin Health and Managing Skin Diseases through Microbiome-Targeted Strategies. Antibiotics (Basel). 2023 Dec 4;12(12):1698. doi: 10.3390/antibiotics12121698. PMID: 38136732; PMCID: PMC10741029.

6. Salli K. Lehtinen MJ. Tiihonen K. Ouwehand AC. Xylitol's Health Benefits beyond Dental Health: A Comprehensive Review. Nutrients. 2019 Aug 6;11(8):1813. doi: 10.3390/nu11081813. PMID: 31390800; PMCID: PMC6723878.

7. Anglenius H. Tiihonen K. Evaluation of xylitol as an agent that controls the growth of skin microbes: Staphylococcus aureus, Staphylococcus epidermidis, and Cutibacterium acnes. Korean J. Microbiol. 2020;56(1):54-58. doi: 10.7845/kjm.2020.0001.

8. Muizzuddin N. Ingrassia M. Marenus KD. Maes DH. Mammone T. Effect of seasonal and geographical differences on skin and effect of treatment with an osmoprotectant: Sorbitol. J Cosmet Sci. 2013 May-Jun;64(3):165-74. PMID: 23752031.

9. Maeda K. Zhou Z. Guo M. Zhang J. Chen L. Yang F. Functional properties and skin care effects of sodium trehalose sulfate. Skin Res Technol. 2024 Apr;30(4):e13666. doi: 10.1111/srt. 13666. PMID: 38606717; PMCID: PMC11010266.

10. Patent WO2022008163A1. Use of trehalose as prebiotic for inhibiting ailment inducing microorganisms. URL: https://patents.google.com/patent/WO2022008163A1/en?oq=WO2022008163

11. Grimshaw. S. G.. Smith. A. M.. Arnold. D. S.. Xu. E.. Hoptroff. M.. & Murphy. B. (2019). The diversity and abundance of fungi and bacteria on the healthy and dandruff affected human scalp. PLoS One. 14(12). e0225796.

**Claims**

1. A hair care and/or scalp care composition, said composition comprising the following components:

   a) rhamnose,
   b) trehalose and
   c) lactobionic acid.

2. The composition of claim 1, wherein said composition has a pH in the range of 4.0 - 9.0, preferably in the range of 5-6, further preferably 5.5-5.7.

3. The composition of claim 1 or claim 2, wherein each of said components is present in said composition in an amount of at least 0.01 % by weight of said composition, preferably 0.02% by weight of said composition, further preferably 0.05% by weight of said composition, further preferably 0.10% by weight of said composition.

4. The composition of claim 1 or claim 2, wherein each of said three components is present in said composition in an amount of a range selected from the following: 0.01-10.00% by weight, 0.01-5.00% by weight, 0.01-3.00% by weight, 0.01-1.00% by weight, 0.01-0.75 % by weight, 0.01-0.50% by weight, 0.02-10.00% by weight, 0.02-5.00% by weight, 0.02-3.00% by weight, 0.02-1.00% by weight, 0.02-0.75 % by weight, 0.02-0.50% by weight, 0.05-10.00% by weight, 0.05-5.00% by weight, 0.05-3.00% by weight, 0.05-1.00% by weight, 0.05-0.75% by weight, 0.05-0.50% by weight, 0.10-10.00% by weight, 0.10-5.00% by weight, 0.10-3.00% by weight, 0.10-1.00% by weight, 0.10-0.75% by weight, 0.10-0.50% by weight.

5. The composition of claim 1 or claim 2, wherein said composition comprises by weight of said composition one of the following:

   i) 0.6% rhamnose, 1.5% trehalose, 1.5% lactobionic acid;
   ii) 0.1% rhamnose, 0.1 % trehalose, 0.1% lactobionic acid;
   iii) 0.2% rhamnose, 0.5% trehalose, 0.5% lactobionic acid.

6. The composition of any of the preceding claims, wherein said composition is formulated for application on a subject's hair and/or scalp.

7. The composition of claim 6, wherein said composition is a formulation selected from the following: liquid, aerosol, suspension, solution, tincture, cream, paste, lotion, ointment, gel, balsam, foam, shampoo and conditioner.

8. Use of the composition of any of claims 1-7 for moisturizing the scalp of a living subject, wherein said subject is preferably a human or an animal.

9. The composition of any of claims 1-7 for use as a medicament.

10. The composition of any of claims 1-7 for promoting health of a scalp or for treating or preventing a scalp disorder of a living subject, wherein said subject is preferably a human or an animal.

11. The composition of any of claims 1-7 for treating or preventing dandruff on the scalp of a living subject, wherein preferably said dandruff is caused by fungi of the *Malassezia* species and/or said subject is a human or an animal.

12. The composition for use of any of claims 1-7 for promoting growth of *Staphylococcus epidermis* and/or *Cutibacterium acnes* on the scalp of a living subject, wherein said subject is preferably a human or an animal.

13. A method of moisturizing a scalp of a living subject or a method for protecting a scalp of a living subject from dehydration, wherein said subject is preferably a human or an animal, said method comprising treating said scalp with a composition of any of claims 1-7.

14. The method of claim 13, wherein said treatment comprises treating said scalp in a leave-on manner with said composition.

15. The use of rhamnose. trehalose and lactobionic acid in the manufacture of composition of any of claims 1-7.

Hydration index for treatment groups

Fig. 1

Elasticity index for treatment groups

Fig. 2

Relative DNA content of *Staphylococcus epidermidis* in samples, normalised to total microbiome by 16 rRNA gene

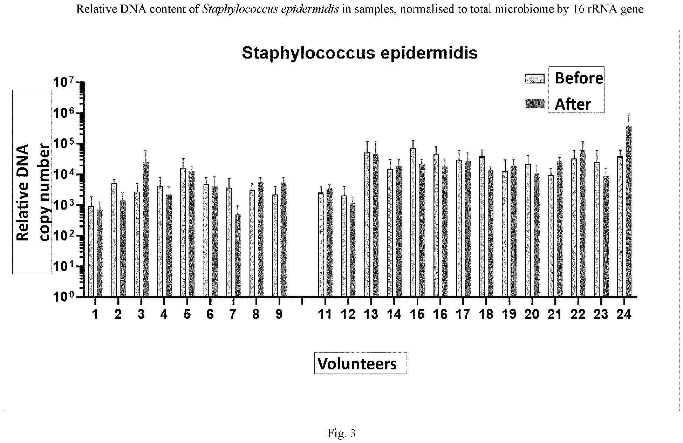

Fig. 3

Change in the relative DNA content of *Staphylococcus epidermidis* in the samples

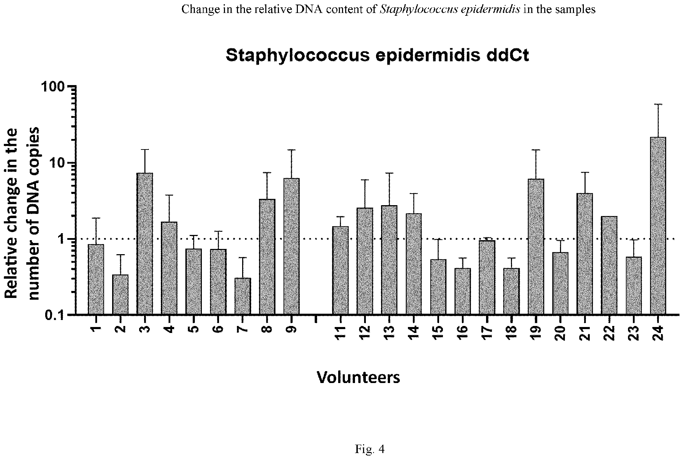

Fig. 4

Relative DNA content of *Cutibacterium acnes* in samples, normalized to total microbiome by 16 rRNA gene

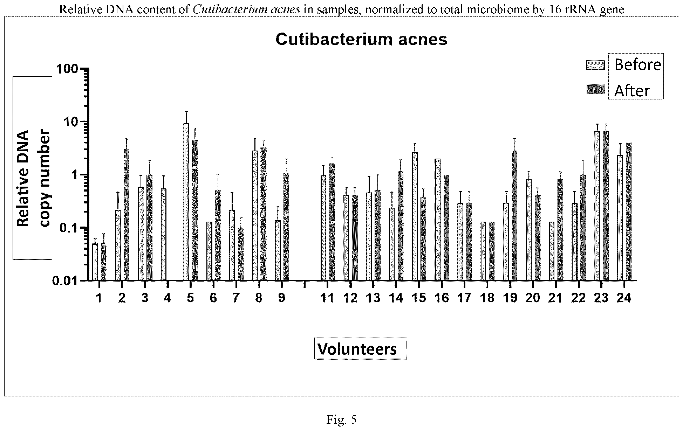

Fig. 5

Change in the relative DNA content of *Cutibacterium acnes* in the samples

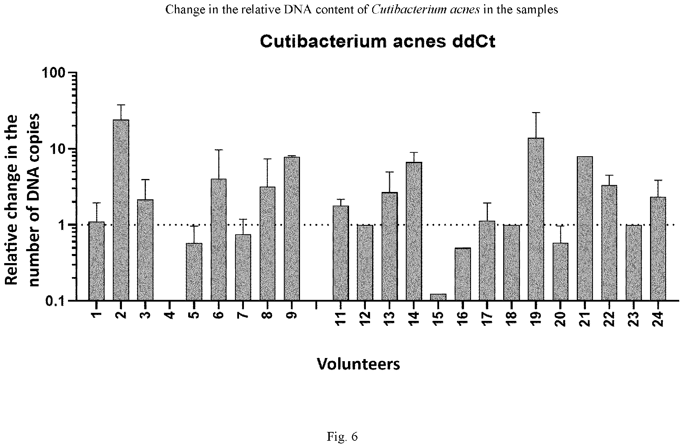

Fig. 6

Relative RNA content of *Staphylococcus epidermidis* in samples, normalized to total microbiome by 16 rRNA gene

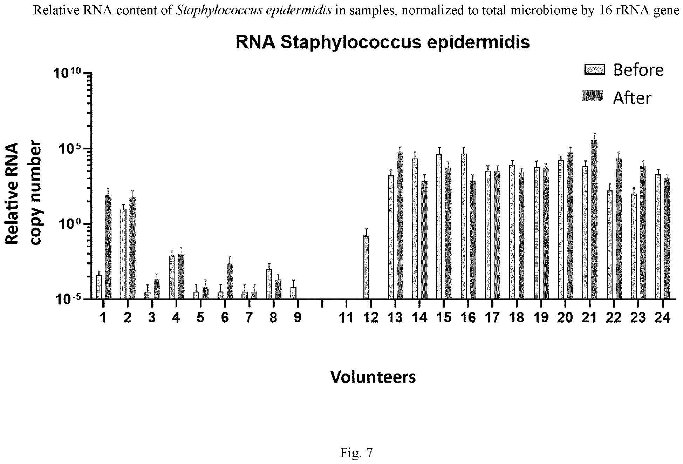

Fig. 7

Change in the relative RNA content of *Staphylococcus epidermidis* in the samples.

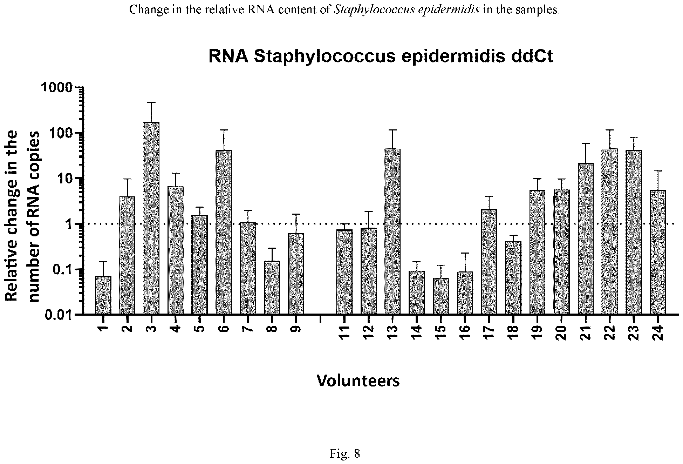

Fig. 8

Relative RNA content of *Cutibacterium acnes* in samples, normalised to total microbiome by 16 rRNA gene

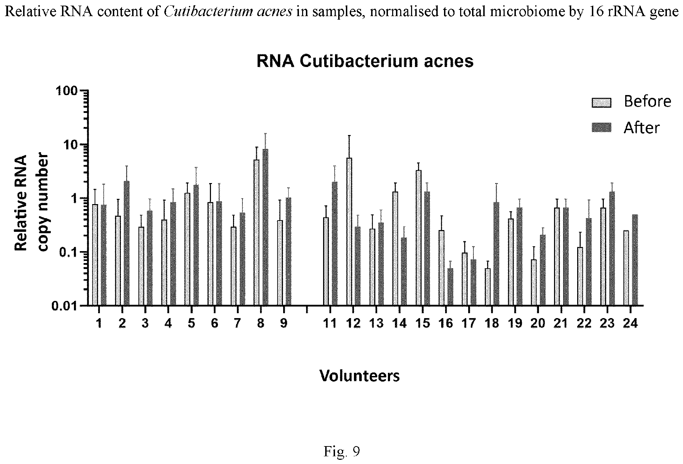

Fig. 9

Change in the relative RNA content of *Cutibacterium acnes* in the samples

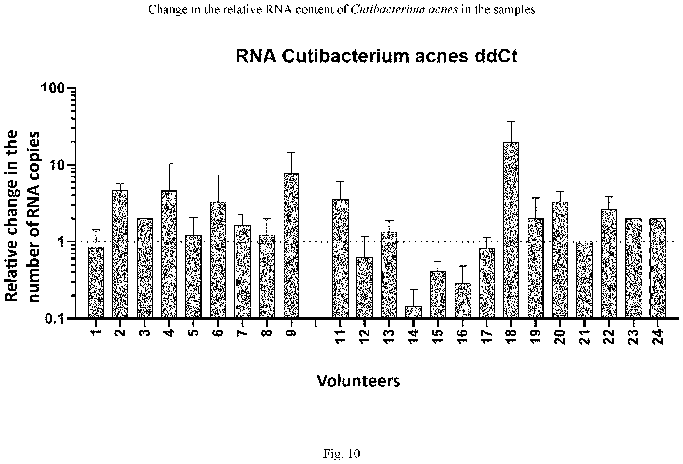

Fig. 10

CFU counts by intercalator qPCR (*S. epidermidis*) as provided in Table 6

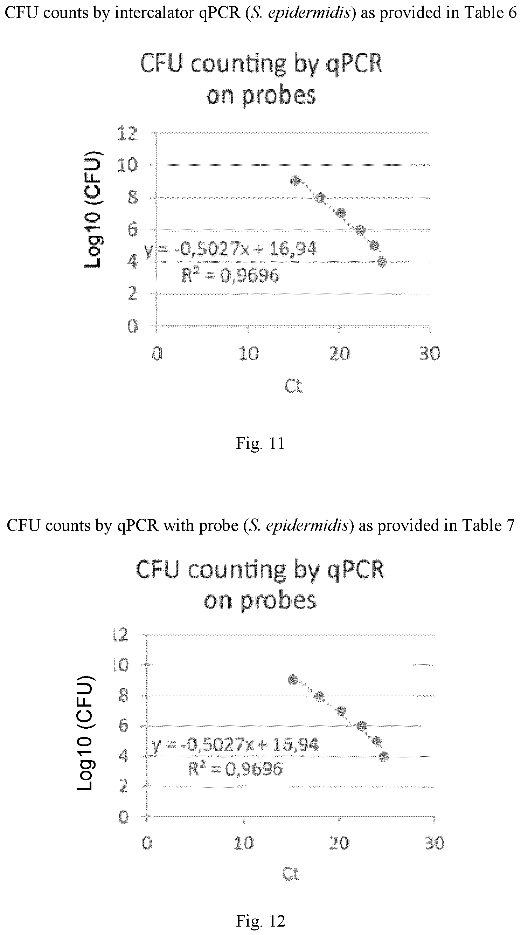

Fig. 11

CFU counts by qPCR with probe (*S. epidermidis*) as provided in Table 7

Fig. 12

Europäisches
Patentamt
European
Patent Office
Office européen
des brevets

# EUROPEAN SEARCH REPORT

**Application Number**

EP 24 21 1339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online] MINTEL; 4 July 2023 (2023-07-04), anonymous: "Energy Lifting Collagen Single Use Essence", XP093262525, Database accession no. 10856398 | 1-4,7,15 | INV. A61K8/60 A61Q5/00 A61Q19/00 |
| Y | * the whole document * | 8-14 | |
| Y,D | WO 2022/008163 A1 (UNILEVER IP HOLDINGS B V [NL]; UNILEVER GLOBAL IP LTD [GB] ET AL.) 13 January 2022 (2022-01-13) | 8-14 | |
| A | * claims; examples * | 5,6 | |
| Y | DATABASE GNPD [Online] MINTEL; 4 January 2018 (2018-01-04), anonymous: "Pinewood Breeze Shampoo", XP093262662, Database accession no. 5354349 * the whole document * | 8-14 | |
| Y | CN 110 944 649 A (ISDIN SA) 31 March 2020 (2020-03-31) | 8-14 | TECHNICAL FIELDS SEARCHED (IPC) A61K A61Q |
| A | * claims; examples * | 5,6 | |
| Y | EP 1 685 843 A1 (YU RUEY J [US]; VAN SCOTT EUGENE J DR [US]) 2 August 2006 (2006-08-02) * claim 105; example 11 * | 8-14 | |

The present search report has been drawn up for all claims

| Place of search | Date of completion of the search | Examiner |
|---|---|---|
| Munich | 25 March 2025 | Mitchell, Gemma |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

.......................................................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 21 1339

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2025

| Patent document cited in search report | | Publication date | Patent family member(s) | | Publication date |
|---|---|---|---|---|---|
| WO 2022008163 | A1 | 13-01-2022 | CN | 115867347 A | 28-03-2023 |
| | | | EP | 4178528 A1 | 17-05-2023 |
| | | | US | 2023270653 A1 | 31-08-2023 |
| | | | WO | 2022008163 A1 | 13-01-2022 |
| CN 110944649 | A | 31-03-2020 | CN | 110944649 A | 31-03-2020 |
| | | | EP | 3658160 A1 | 03-06-2020 |
| | | | ES | 2884787 T3 | 13-12-2021 |
| | | | PT | 3658160 T | 26-08-2021 |
| | | | US | 2020222445 A1 | 16-07-2020 |
| | | | WO | 2019020822 A1 | 31-01-2019 |
| EP 1685843 | A1 | 02-08-2006 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014053625 A2
- WO 2022008163 A1

**Non-patent literature cited in the description**

- *CHEMICAL ABSTRACTS*, 3615-41-6
- *CHEMICAL ABSTRACTS*, 10030-85-0
- *CHEMICAL ABSTRACTS*, 99-20-7
- *CHEMICAL ABSTRACTS*, 96-82-2
- **SAXENA R.** ; **MITTAL P.** ; **CLAVAUD C.** ; **DHAKAN DB** ; **HEGDE P.** ; **VEERANAGAIAH MM** ; **SAHA S.** ; **SOUVERAIN L.** ; **ROY N.** ; **BRETON L.** Comparison of Healthy and Dandruff Scalp Microbiome Reveals the Role of Commensals in Scalp Health. *Front Cell Infect Microbiol.*, 04 October 2018, vol. 8, 346
- **SAXENA. R.** ; **MITTAL. P.** ; **CLAVAUD. C. et al.** Longitudinal study of the scalp microbiome suggests coconut oil to enrich healthy scalp commensals. *Sci Rep*, 2021, vol. 11, 7220, https://doi.org/10.1038/s41598-021-86454-1
- **POLAK-WITKA K.** ; **RUDNICKA L.** ; **BLUME-PEYTAVI U.** ; **VOGT A.** The role of the microbiome in scalp hair follicle biology and disease. *Exp Dermatol.*, 15 May 2019, vol. 29 (3), 286-294
- **GALLO G.** ; **GROSSI U.** ; **DI TANNA GL.** ; **SANTORO GA.** ; **DE PAOLA G.** ; **CLERICO G.** ; **REALIS LUC A.** ; **TROMPETTO M.** ; **SAMMARCO G.** Short-Term Outcomes of Polycarbophil and Propionibacterium acnes Lysate Gel after Open Hemorrhoidectomy: A Prospective Cohort Study. *J Clin Med.*, 10 December 2020, vol. 9 (12), 3996

- **AL-SMADI K.** ; **LEITE-SILVA VR.** ; **FILHO NA.** ; **LOPES PS.** ; **MOHAMMED Y.** Innovative Approaches for Maintaining and Enhancing Skin Health and Managing Skin Diseases through Microbiome-Targeted Strategies. *Antibiotics (Basel)*, 04 December 2023, vol. 12 (12), 1698
- **SALLI K.** ; **LEHTINEN MJ.** ; **TIIHONEN K.** ; **OUWE-HAND AC.** Xylitol's Health Benefits beyond Dental Health: A Comprehensive Review. *Nutrients*, 06 August 2019, vol. 11 (8), 1813
- **ANGLENIUS H.** ; **TIIHONEN K.** Evaluation of xylitol as an agent that controls the growth of skin microbes: Staphylococcus aureus, Staphylococcus epidermidis, and Cutibacterium acnes. *Korean J. Microbiol.*, 2020, vol. 56 (1), 54-58
- **MUIZZUDDIN N.** ; **INGRASSIA M.** ; **MARENUS KD.** ; **MAES DH.** ; **MAMMONE T.** Effect of seasonal and geographical differences on skin and effect of treatment with an osmoprotectant: Sorbitol. *J Cosmet Sci.*, May 2013, vol. 64 (3), 165-74
- **MAEDA K.** ; **ZHOU Z.** ; **GUO M.** ; **ZHANG J.** ; **CHEN L.** ; **YANG F.** Functional properties and skin care effects of sodium trehalose sulfate. *Skin Res Technol.*, April 2024, vol. 30 (4), e13666
- **GRIMSHAW. S. G.** ; **SMITH. A. M.** ; **ARNOLD. D. S.** ; **XU. E.** ; **HOPTROFF. M.** ; **MURPHY. B.** The diversity and abundance of fungi and bacteria on the healthy and dandruff affected human scalp. *PLoS One*, 2019, vol. 14 (12), e0225796